# EUROPEAN PATENT SPECIFICATION

(11) **EP 1 373 202 B1**
(45) Date of publication and mention of the grant of the patent: **27.04.2005**
(21) Application number: 02734878.8
(22) Date of filing: 22.01.2002
(51) Int. Cl.: C07D 207/34, C07D 405/06

(54) **PREPARATION OF NON-CRYSTALLINE ATORVASTATIN CALCIUM**
HERSTELLUNG VON NICHTKRISTALLINEM ATORVASTATIN?CALCIUM
PREPARATION DE CALCIUM D'ATORVASTATINE NON CRISTALLIN

(30) Priority: 23.01.2001 SI 200110010
(43) Date of publication of application: 02.01.2004
(73) Proprietor: Lek Pharmaceutical and Chemical Co. D.D., 1526 Ljubljana (SI)
(72) Inventor: SORSAK, Gorazd, 2325 Kidricevo (SI)
(74) Representative: Leson, Thomas Johannes Alois, Dipl.-Ing.
(86) International application number: PCT/IB2002/000161
(87) International publication number: WO 2002/059087

(56) References cited:
- WO-A-00/71116
- WO-A-97/03960
- BAUMANN K L ET AL: "THE CONVERGENT SYNTHESIS OF CI-981, AN OPTICALLY ACTIVE, HIGHLY POTENT, TISSUE SELECTIVE INHIBITOR OF HMG-COA REDUCTASE" TETRAHEDRON LETTERS, ELSEVIER SCIENCE PUBLISHERS, AMSTERDAM, NL, vol. 33, no. 17, 21 April 1992 (1992-04-21), pages 2283-2284, XP000608147 ISSN: 0040-4039

## Description

### Technical field

Atorvastatin calcium, the substance known by the chemical name [(R-(R*,R*)]-2-(4-fluorophenyl)-b,d-dihydroxy-5-(1-methylethyl)-3-phenyl-4- (phenylamino)carbonyl -1H-pyrrole-1-heptanoic acid hemi calcium salt is known as HMG-CoA reductase inhibitor and is used as an antihypercholesterolemic agent. Processes for the preparation of atorvastatin and key intermediates are disclosed in the United States Patent Numbers: 5,003,080; 5,097,045; 5,103,024; 5,124,482; 5,149,837; 5,155,251; 5,216,174; 5,245,047; 5,248,793; 5,280,126; 5,342,952 and 5,397,792. Atorvastatin is usually prepared as its calcium salt since it enables atorvastatin to be conveniently formulated in the pharmaceutical formulations, for example, in tablets, capsules, powders and the like for oral administration.

Atorvastatin calcium can exist in an amorphous form or in one of the crystalline forms (Form I, Form II, Form III and Form IV), which are disclosed in the PCT patent applications WO-A-97/3958 and WO-A-97/3959. It is known that the amorphous forms in a number of pharmaceutical substances exhibit different dissolution characteristics and bioavailability patterns compared to the crystalline forms (Konno T., *Chem*. *Pharm. Bull*., 1990, 38: 2003-2007). For some therapeutic indications the bioavailability is one of the key parameters determining the form of the substance to be used in a pharmaceutical formulation. Since processes for the crystallization and the preparation, respectively, of the amorphous substance are sometimes difficult to be performed, and as a product afford amorphous-crystalline mixtures, that is, a crystalline form instead of an amorphous form, there is a constant need for the processes which enable the preparing a non-crystalline form without simultaneous formulation of crystalline forms, that is, which will enable the conversion of the crystalline form into the non-crystalline form.

Atorvastatin calcium is the substance which is very slightly water-soluble, and it has been found that the crystalline forms are less readily soluble than the amorphous form which may cause problems in the bioavailability of atorvastatin in the body. It has been found that the production of amorphous atorvastatin calcium according to the previously disclosed processes was not consistently reproducible, therefore a process has been developed for converting the crystalline forms of atorvastatin calcium (formed in the synthesis of atorvastatin) to the amorphous form. The process is described in the PCT patent application WO-A-97/3960 and comprises dissolving the crystalline form of atorvastatin calcium in a non-hydroxylic solvent and after removal of the solvent affords amorphous atorvastatin calcium. The preferred non-hydroxylic solvent is selected from the group consisting of tetrahydrofuran, and a mixture of tetrahydrofuran and toluene. The disadvantage of the above process is primarily use of non-nature-friendly solvents. A similar process is described in the PCT patent application WO-A-00/71116 and comprises dissolving the crystalline form of atorvastatin calcium in a non-hydroxylic solvent, such as, for example, tetrahydrofuran. To a solution of atorvastatin calcium is added a nonpolar organic solvent, or a solution of atorvastatin calcium is added to a nonpolar organic solvent to allow atorvastatin calcium to precipitate. The formed precipitate is filtered off.

Synthesis of atorvastatin calcium is demanding and accordingly the cost of the finished product is high. Therefore, it was an object to minimize the number of synthesis steps in the process for the preparation of atorvastatin calcium and in this manner to improve the yield.

The present invention provides the conversion of an intermediate compound having the formula (I) shown below into non-crystalline, in particular amorphous, atorvastatin calcium without the need of prior formation of atorvastatin lactone and atorvastatin calcium in the form of crystals or a mixture of crystals of amorphous and crystalline form of atorvastatin calcium. In a further aspect, the present invention also provides the conversion of atorvastatin in the form of lactone into non-crystalline, in particular amorphous, atorvastatin calcium without intermediate formation of atorvastatin calcium in the form of crystals or a mixture of amorphous and crystalline form. In a still further aspect, the present invention also provides a process for the preparation of a pharmaceutical formulation containing atorvastatin calcium which had been prepared directly in the non-crystalline, in particular in the amorphous form.

Accordingly, the present invention in the first aspect provides a novel process for the direct preparation of non-crystalline atorvastatin calcium from the following intermediate compound without the prior transformation into atorvastatin lactone or atorvastatin calcium in a crystalline form, respectively, which process comprises the following steps:
a) providing a solution containing an intermediate compound having the following formula (I) in a non-hydroxylic solvent: 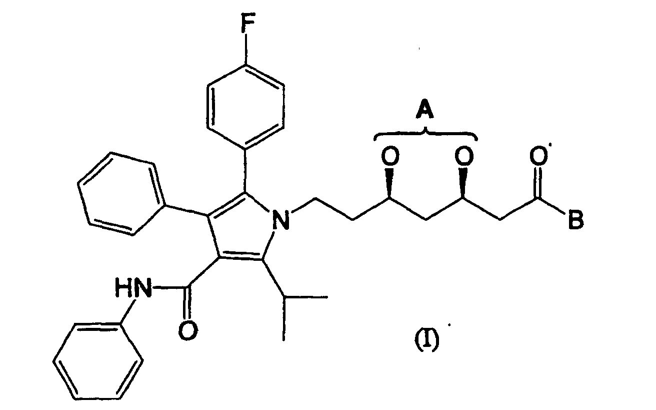 wherein A denotes a common protection group or separate protection groups for the dihydroxy group and B denotes a carboxylic acid protection group;
b) carrying out deprotection of the dihydroxy group;
c) carrying out deprotection of the carboxylic acid protection group;
   wherein the order of steps b) and c) can be reversed;
d) concentrating the solution to about half of the initial volume or lower;
e) adding water in excess of the volume of the concentrated solution;
f) adding, using about the same or a higher volume than the water volume added in step e), a solvent which is slightly miscible or immiscible with water and in which atorvastatin calcium is insoluble or practically insoluble;
g) optionally performing a mixing operation, and separating the two phases;
h) neutralizing the aqueous phase;
i) converting the dihydroxy carboxylic acid form of atorvastatin to a pharmaceutically acceptable salt form; and
j) forming a precipitate of the atorvastatin being converted in said pharmaceutically acceptable salt form.

The preparation of the intermediate compound of formula (I) is described in EP 0 330 172 and WO 99/20492, both documents being incorporated herein by reference. The intermediate compound preferably has the following formula (II): 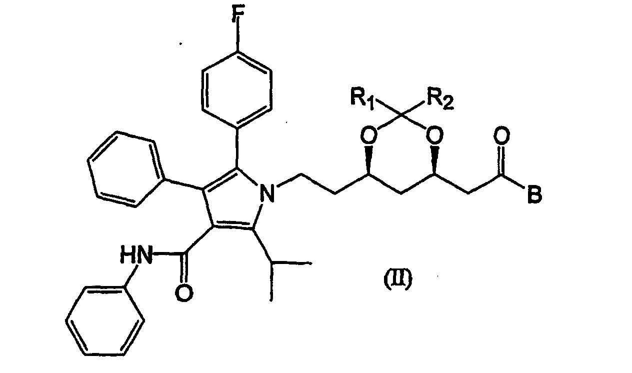 wherein R₁ and R₂ are independently hydrogen, alkyl of from one to three carbon atoms, or phenyl, or R₁ and R₂ are taken together as (-CH₂)ₙ- wherein n is 4 or 5, and
B is
a) O-R₃ wherein R₃ is
   - straight chain or branched chain alkyl of from one to eight carbon atoms, preferably tert-butyl, tert-amyl or α,α-dimethylbenzyl, or
   - a three- to six-membered cycloalkyl group,
b) a group of the formula: 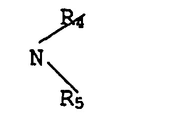 wherein R₄ and R₅ are independently alkyl of from one to ten carbon atoms, cycloalkyl of from three to seven carbon atoms such as cyclopropyl, cyclobutyl, cyclopentyl and cyclohexyl, aryl or aralkyl such as benzyl or phenyl, or R₄ and R₅ together form a four to six member hydrocarbon linkage group optionally containing one or more hetero atoms such as *O* and optionally being substituted by an alkyl of from one to four carbon atoms, e. g. -(CH₂)₄-, -(CH₂)₅-, -CH(R₆)-(CH₂)₃-, -CH(R₆)-(CH₂)₄-, -CH(R₆)-(CH₂)₂-CH(R₆)-, -CH (R₆)-(CH₂)₃-CH(R₆)-, -CH₂-CH₂-O-CH₂-CH₂-, -CH (R₆)-CH₂-O-CH₂-CH₂- and -CH(R₆)-CH₂-O-CH₂-CH(R₆)-, wherein R₆ is alkyl of from one to four carbon atoms.

A particular example for the intermediate compound used as the starting material is the compound having the following formula (III):

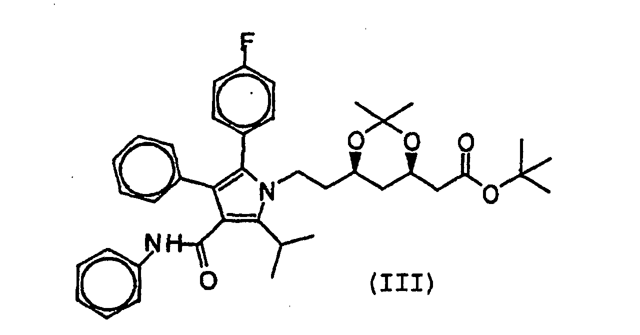

In the second aspect, the present invention further relates to the process for the conversion of atorvastatin in the form of lactone into a non-crystalline form of atorvastatin calcium. In this alternative process, atorvastatin in the form of lactone is provided in a non-hydroxylic solvent; a reaction for opening the lactone ring is performed; and then the steps as defined by steps d) to j) specified above in connection with the first aspect of the invention are carried out.

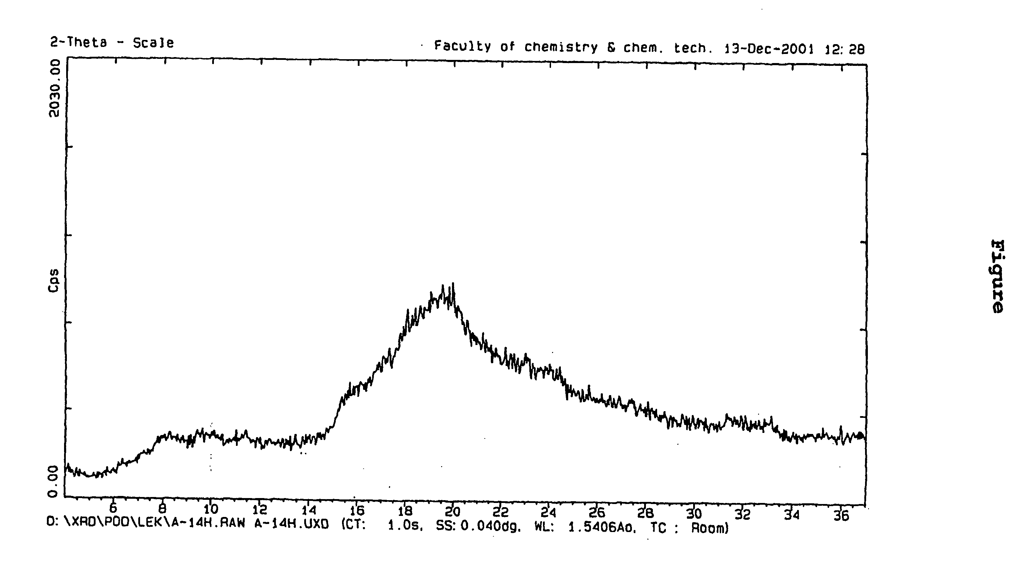

### Brief description of the Figure

The Figure shows an X-ray powder diffractogram of atorvastatin calcium obtained with a process according to the present invention.

The present invention is described in more detail by referring to the following embodiments.

According to the process, the intermediate compound of formula (I), especially that of formula (II) and in particular that of formula (III) being defined by more specific protecting groups, is provided in solution. The solution may be provided in the course of the synthesis of the intermediate compound, or the compound may be dissolved in an appropriate amount, for example 100 to 300 ml (maximum to concentration of the intermediate to 80 g/liter), of a non-hydroxylic solvent such as, for example, tetrahydrofuran, 1,4-dioxane, acetone, ethyl acetate or a mixtures of this solvents; or mixtures of mentioned solvents with toluene, n-heptane, n-hexane, acetonitrile in the volume ratio between 1:0.01 to 1:1.0. Then, the deprotection of the hydroxyl groups in the side-chain (in the 3- and 5-positions) of the intermediate compound is performed, which can conveniently be done by the addition of an acid such as mineral acids, for example diluted hydrochloric acid or sulfuric acid, trifluoroacetic acid, formic acid, propanic acid, para-toluenesulfonic acid. The amount of the added acid to intermediate compound lies in a molar ratio of from between 1:0.05 to 1:0.2 (for monoprotonic acids), preferably between 1:0.09 and 1:0.1. The resulting solution is kept, preferably while being mixed by stirring, agitating or shaking the solution, at a temperature of from 5 to 40°C, preferably at a room temperature so that the intermediate compound (I), (II) or (III), respectively, is no longer detectable by thin-layer chromatography (TLC). Then, the deprotection of the carboxylic acid group (removal of moiety B such as R₃, e.g., tert-butyl), is carried out, which can conveniently be done by adding an appropriate base such as alkali metal hydroxide or alkaline earth metal hydroxide, for example sodium hydroxide, potassium hydroxide, lithium hydroxide, barium hydroxide and the like, sodium or potassium hydroxide being preferred, to the solution to adjust the pH of the solution to a range of from 8 to 13, preferably from 9 to 12. The resulting solution is kept, preferably while being mixed by stirring, agitating or shaking the solution, at a temperature of from 5 to 40°C, preferably at room temperature so that hydroxyl group deprotected, yet carboxylic acid group protected intermediate compound is no longer detectable by thin-layer chromatography (TLC).

The solution is then concentrated, for example by evaporation in vacuo, to about half of the initial volume or lower, preferably between 15 and 50% of the initial volume and more preferably to about 1/4 of the initial volume. The concentrated solution is diluted with a volume of water in excess of the volume of the concentrated solution, preferably in 0.6 to 3-fold of the volume of the concentrated solution. To this solution is added, using about the same or a higher volume than the previously added water volume, preferably a 1 to 5-fold and more preferably 2 to 3-fold of the previously added water volume, of a solvent which is slightly miscible or immiscible with water and in which atorvastatin calcium is insoluble or practically insoluble. Examples of suitable solvents include hexane, heptane, cyclohexane, ether, diisopropyl ether or the like. Preferably, the resulting solution is vigorously mixed, for example by stirring, agitating or shaking, and subsequently the phases are separated. Then, the aqueous phase is preferably rapidly stirred, agitated or shaken while an acid, e.g. a mineral acid as mentioned above such as hydrochloric acid, is carefully added to neutralize the solution, preferably adjusting the pH of the aqueous phase to a range of from 6.5 to 8, more preferably to a pH of from 6.8 to 7.5.

Then, the dihydroxy carboxylic acid form of atorvastatin thus obtained is converted to a pharmaceutically acceptable salt form. The most preferred salt form is the calcium salt. This may be carried out by heating the resulting neutralized aqueous solution to a temperature of from 30 to 40°C, preferably at about 35°C. To this solution, which is rapidly mixed by stirring, agitation or shaking, is added a 0.05 to 0.5M, preferably 0.1 to 0.3M aqueous solution of the corresponding salt which is correspondingly preheated to 30 to 40°C, preferably at about 35°C. In order to obtain the preferred calcium salt form of atorvastatin, a suitable calcium salt, preferably calcium acetate, calcium citrate, calcium oxalate, calcium chloride or calcium iodide, is used. The amount of the added salt to intermediate starting compound preferably lies in the molar ratio between 1:1 and 1:1.55, preferably 1:1.13 and 1:1.135. After the completed addition, the mixture is preferably kept, suitably under a mixing operation like stirring, agitating or shaking, for a suitable period, for example for 0.5 to 3 hours and preferably for about 1 to 2 hours, at a temperature between 10 and 30°C, preferably between 20 and 25°C.

Then, a precipitate of the atorvastatin being converted in said pharmaceutically acceptable salt form is formed. To this end, the resulting solution may be cooled to a lower temperature, for example to a temperature of from 2 to 15 °C, preferably from 4 to 10°C. In place of cooling the solution, atorvastatin calcium may also be precipitated by the addition of a water-miscible organic solvent in which atorvastatin calcium is slightly soluble or practically insoluble.

As a further alternative, atorvastatin calcium may be precipitated by concentrating the solution, for example, in a vacuum evaporator.

To give atorvastatin calcium in the desired non-crystalline form, the formed precipitate may be obtained by appropriate means and, thus, may be filtered, rinsed with water and dried.

In case the starting substance is atorvastatin in the form of lactone, the lactone compound (which can be produced according to the references mentioned above) is correspondingly provided in solution. Likewise, the solution may be provided in the course of the synthesis of the lactone compound, or the lactone compound may be dissolved in an appropriate amount, for example 100 to 300 ml of a non-hydroxylic solvent such as, for example, tetrahydrofuran.

Then, a reaction to open the lactone ring is performed, which is suitably done by adding a base, for example an alkali metal or alkaline earth metal hydroxide as mentioned above such as NaOH. The amount of the added base to lactone lies in a molar ratio between 1:0.2 and 1:0.6, preferably 1:0.29 and 1:0.57. The resulting solution is heated to an appropriate temperature, suitably to 40 to 60°C and perferably to about 50°C, and maintained at this temperature for a suitable period until the lactone form is no longer detectable by TLC.

Subsequently, the solution is concentrated and further processed as described above for the preparation of the non-crystalline substance from intermediate compound (I) (see steps d) to j) described above).

According to the third aspect of the present invention, the process for the preparation of a pharmaceutical formulation containing atorvastatin calcium in a non-crystalline form comprises preparing atorvastatin calcium in a non-crystalline form from either intermediate compound having the formula (I) (more specifically the formulae (II) or (III)) or from the lactone form, and mixing the thus prepared non-crystalline atorvastatin calcium with a pharmaceutically acceptable carrier in a conventional manner. Preferably, a non-crystalline atorvastatin in the calcium salt form is prepared. The pharmaceutical formulation is generally solid in the form of tablets, capsules, powders and the like for oral administration.

The pharmaceutical formulation thus prepared may include, in addition to the thus directly prepared non-crystalline atorvastatin calcium, in particular the calcium hemisalt, one or more fillers, such as microcrystalline cellulose, lactose, sugars, starches, modified starch, mannitol, sorbitol and other polyols, dextrin, dextran and maltodextrin, calcium carbonate, calcium phosphate and/or hydrogen phosphate, sulphate, one or more binders, such as lactose, starches, modified starch, dextrin, dextran and maltodextrin, microcrystalline cellulose, sugars, polyethylene glycols, hydroxypropyl cellulose, hydroxypropyl methylcellulose, ethylcellulose, hydroxyethyl cellulose, methylcellulose, carboxymethyl cellulose, gelatin, acacia gum, tragacanth, polyvinylpyrrolidone, magnesium aluminium silicate, one or more disintegrating agents such as croscarmellose sodium, cross-linked polyvinylpyrrolidone, cross-linked carboxymethyl starch, starches and microcrystalline cellulose, magnesium aluminium silicate, polyacrylin potassium, one or more different glidants such as magnesium stearate, calcium stearate, zinc stearate, calcium behenate, sodium stearyl fumarate, talc, magnesium trisilicate, stearic acid, palmitic acid, carnauba wax, silicon dioxide, one or more buffering agents such as sodium or potassium citrate, sodium phosphate, dibasic sodium phosphate, calcium carbonate, hydrogen phosphate, phosphate, sulphate, sodium or magnesium carbonate, sodium ascorbinate, benzoate, sodium or potassium hydrogen carbonate, lauryl sulphate, or mixtures of such buffering agents.

If required, the formulation may also include surfactants and other conventional components for solid, pharmaceutical formulations such as coloring agents, lakes, aromas and adsorbents. As surfactants the following may be used: ionic surfactants, such as sodium lauryl sulphate or non-ionic surfactants such as different poloxamers (polyoxyethylene and polyoxypropylene copolymers), natural or synthesized lecithins, esters of sorbitan and fatty acids (such as Span®, manufactured by Atlas Chemie), esters of polyoxyethylenesorbitan and fatty acids (such as Tween®, manufactured by Atlas Chemie), polyoxyethylated hydrogenated castor oil (such as Cremophor®, manufactured by BASF), polyoxyethylene stearates (such as Brij®, manufactured by Atlas Chemie), dimethylpolysiloxane or any combination of the above mentioned surfactants.

If the pharmaceutical formulation is in the form of coated tablets, the coating may be prepared from at least one film-former such as hydroxypropyl methylcellulose, hydroxypropyl cellulose, at least from one plasticizer such as polyethylene glycols, dibutyl sebacate, triethyl citrate, and other pharmaceutical auxiliary substances conventional for film coatings, such as pigments, fillers and others.

The pharmaceutical formulation may be prepared by conventional methods known to those skilled in the art.

The present invention is illustrated but in no way limited by the following examples.

### EXAMPLES

### Example 1

4.37 g (0.0067 mol) of the compound III were dissolved in 200 ml of tetrahydrofuran, 15 ml of 10% HCl was added and the solution was stirred at room temperature for 15 hours. To this solution 3.6 g (0.090 mol) of solid NaOH were added and stirred for additional 30 hours. The solution was concentrated (evaporated by vacuum) to 50 ml. 50 ml of water and 80 ml of hexane were added. The phases were separated and to the rapidly agitated aqueous phase 5M HCl was added carefully to a pH to 7.0-7.5. The solution is heated to 35°C and 0.76 g (0.0043 mol) Ca (OAc)₂ x H₂O in 20 ml of water, preheated to 35°C was added to the agitated solution. After the completed addition, the solution is stirred for additional 1 hour at room temperature and then placed in a refrigerator for 2 hours. The formed precipitate was filtered, rinsed with water (2 x 20 ml) and dried at 40°C for 18 hours to give 3.75 g of the non-crystalline product.

### Example 2

3.00 g of the compound III were dissolved in 140 ml of tetrahydrofuran, 10 ml of 10% HCl were added and the solution was stirred at room temperature. To this solution 3.6 g of solid NaOH were added and the solution was stirred for 30 hours. The solution was concentrated (evaporated by vacuum) to 1/4 - 1/5 of the initial volume. Then the same amount of water, and 1.6-fold amount of hexane as the volume of the remaining concentrated solution were added. The phases were separated and to the rapidly agitated aqueous phase 5M HCl was added carefully to a pH to 7.0. The solution is heated to 35°C and 0.76 g Ca(OAc)₂ x H₂O in 20 ml of water, preheated to 35°C was added to the agitated solution. After the completed addition, the solution is stirred for additional 1 hour at room temperature and then placed in a refrigerator for 2 hours. The formed precipitate was filtered, rinsed with water and dried at 40°C for 18 hours to give 2.23 g of the non-crystalline atorvastatin calcium.

The obtained non-crystalline atorvastatin calcium has an X-ray powder diffractogram substantially as shown in the Figure. The X-ray powder diffraction pattern was collected on a Philips PW1710 diffractometer in reflection geometry. The instrument is regularly calibrated with the silicon standard. The sample was not ground before the measurement. Standard Philips back-loading sample holder was used. Sample storage, mounting and data collection were done at room temperature.
Instrumental parameters: CuKα radiation (30mA, 40kV, λ=1.5406Å), variable divergence slit (approx. 12 x 16mm irradiated area), 0.4mm receiving slit, graphite monochromator on the secondary side, scintillation counter.
Data collection parameters: 2θ range from 4 to 37°, step scan mode in steps of 0.04°2θ, integration time 1s at each step.

## Claims

1. A process for the preparation of atorvastatin in a non-crystalline form, which comprises:
a) providing a solution containing an intermediate compound having the following formula (I) in a non-hydroxylic solvent: 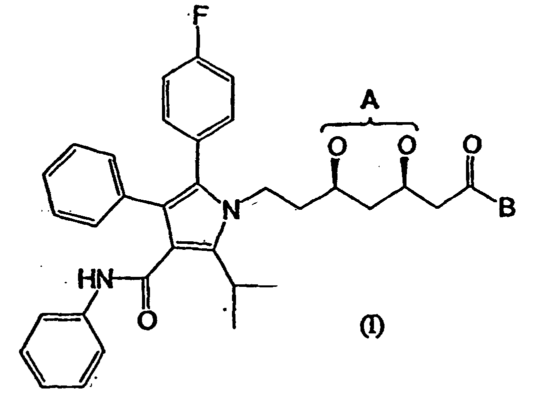 wherein A denotes a common protection group or separate protection groups for the dihydroxy group and B denotes a carboxylic acid protection group;
b) carrying out deprotection of the dihydroxy group;
c) carrying out deprotection of the carboxylic acid protection group;
wherein the order of steps b) and c) may be reversed;
d) concentrating the solution to about half of the initial volume or lower;
e) adding water in excess of the volume of the concentrated solution;
f) adding, using about the same or a higher volume than the water volume added in step e), a solvent which is slightly miscible or immiscible with water and in which atorvastatin calcium is insoluble or practically insoluble;
g) optionally performing a mixing operation, and separating the two phases;
h) neutralizing the aqueous phase;
i) converting the dihydroxy carboxylic acid form of atorvastatin to a pharmaceutically acceptable salt form; and
j) forming a precipitate of the atorvastatin being converted in said pharmaceutically acceptable salt form.

2. The process according to claim 1, wherein a solution containing the intermediate compound having the following formula (II) is provided in step a): 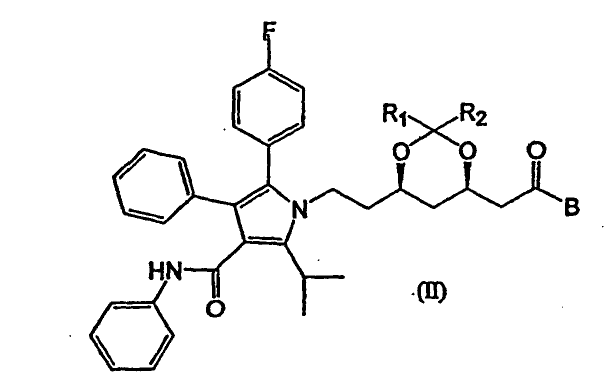 wherein R₁ and R₂ are independently hydrogen, alkyl of from one to three carbon atoms, or phenyl, or R₁ and R₂ are taken together as (-CH₂)ₙ- wherein n is 4 or 5,
B is
a) O-R₃ wherein R₃ is
- straight chain or branched chain alkyl of from one to eight carbon atoms (R₃ is tert-butyl, tert-amyl or α, α-dimethylbenzyl), or
- a three- to six-membered cycloalkyl group,
b) a group of the formula: 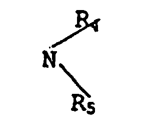
wherein R₄ and R₅ are independently alkyl of from one to ten carbon atoms, cycloalkyl of from three to seven carbon atoms, aryl or aralkyl, or R₄ and R₅ together form a four to six member hydrocarbon linkage group optionally containing one or more hetero atoms and optionally being substituted by an alkyl of from one to four carbon atoms.

3. The process according to claim 1, wherein a solution containing the intermediate compound having the following formula (III) is provided in step a):

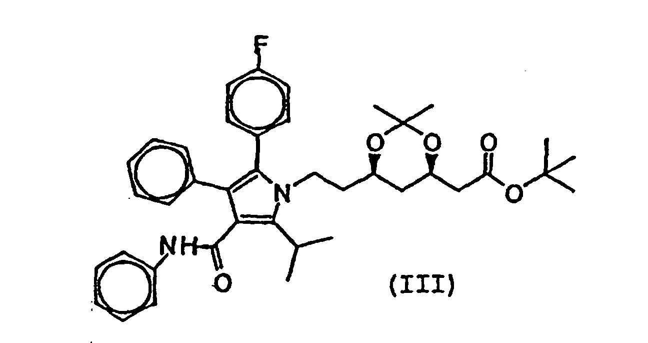

4. The process according to any one of claims 1 to 3, wherein the deprotection of the dihydroxy group in step b) is carried out by adding an acid and keeping or mixing the solution at a temperature of from 5 to 40°C.

5. The process according to any one of claims 1 to 3, wherein the deprotection of the carboxylic acid protection group in step c) is carried out by adding a base to adjust the pH of the solution to a range of from 8 to 13 and keeping or mixing the solution at a temperature of from 5 to 40°C.

6. The process according to any one of claims 1 to 3, wherein in step d) the solution is concentrated to 15 to 50% of the initial volume.

7. The process according to any one of claims 1 to 3, wherein water is added in step e) in 0.6 to 3-fold relative to the volume of the concentrated solution.

8. The process according to any one of claims 1 to 3, wherein water is added in step e) in 0.6 to 1.5-fold relative to the volume of the concentrated solution.

9. The process according to any one of claims 1 to 3, wherein said solvent is added in step f) at an amount of 1 to 5-fold of the water volume previously added in step e).

10. The process according to any one of claims 1 to 3, wherein neutralizing the aqueous phase in step h) is carried out by adding an acid to the aqueous phase to adjust its pH to a range of from 6.5 to 8.

11. The process according to any one of claims 1 to 3, wherein the conversion in step i) is carried out by heating the neutralized aqueous solution to a temperature of from 30 to 40°C, and then adding an aqueous solution of the corresponding salt being preheated to 30 to 40°C.

12. The process according to any one of claims 1 to 3, or 11, wherein, after the addition of a corresponding salt, keeping the solution under a mixing operation at a temperature in the range of from 10 to 30°C.

13. The process according to any one of claims 1 to 3, 11 or 12, wherein the salt is a calcium salt.

14. The process according to any one of claims 1 to 3, wherein the precipitation step j) comprises adjusting the temperature of the solution to a range of from 2 to 15°C to afford a precipitate of non-crystalline atorvastatin in the pharmaceutically acceptable salt form.

15. The process according to any one of claims 1 to 3, wherein the precipitation step j) comprises adding an organic solvent which is miscible with water and in which atorvastatin is practically insoluble or insoluble.

16. The process according to any one of claims 1 to 3, wherein the precipitation step j) comprises concentrating the solution.

17. The process according to any one of claims 1 to 3, 14 to 16, which comprises a further step k) by filtering off the formed precipitate, rinsing the precipitate with water, and drying the precipitate to give the non-crystalline atorvastatin calcium.

18. A process for the preparation of atorvastatin calcium in a non-crystalline form, which comprises:
- providing atorvastatin in the form of lactone in a non-hydroxylic solvent;
- performing a reaction for opening the lactone ring; and then
- carrying out the steps as defined by steps d) to j) set forth in claim 1.

19. The process according to claim 18, wherein the lactone ring is opened by adding a base and heating the solution to a temperature of from 40 to 60°C.

20. The process according to claim 18, wherein any one of the process steps as defined in claims 6 to 17 in connection with steps d)-f) and h)-k) are carried out.

21. A process for the preparation of a pharmaceutical formulation containing atorvastatin in a non-crystalline form, comprising preparing atorvastatin in a non-crystalline form in accordance with claim 1 or claim 18 and mixing it with a pharmaceutically acceptable carrier.

22. The process according to claim 21, wherein non-crystalline atorvastatin in the calcium salt form is prepared.

## Patentansprüche

1. Verfahren zur Herstellung von Atorvastatin in einer nicht-kristallinen Form, welches das Folgende umfasst:
a) Bereitstellen einer Lösung mit einer intermediären Verbindung mit der folgenden Formel (I) in einem nicht-hydroxylischen Lösungsmittel: 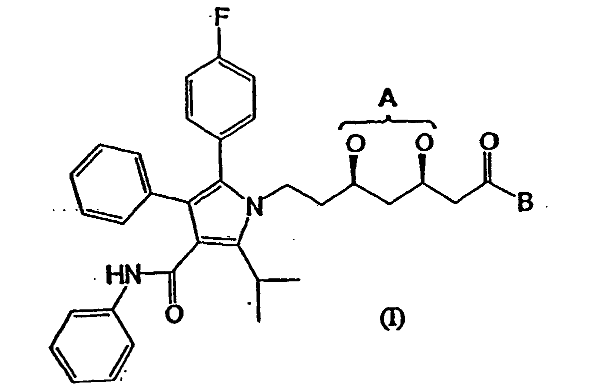 worin A eine gemeinsame Schutzgruppe oder separate Schutzgruppen für die Dihydroxygruppe bedeutet und B eine Carbonsäureschutzgruppe bedeutet;
b) Durchführen des Entschützens der Dihydroxygruppe;
c) Durchführen des Entschützens der Carbonsäureschutzgruppe; wobei die Reihenfolge der Schritte b) und c) umgekehrt werden kann;
d) Konzentrieren der Lösung auf die Hälfte oder weniger des anfänglichen Volumens;
e) Zugeben von Wasser im Überschuss des Volumens der konzentrierten Lösung;
f) Zugeben eines Lösungsmittels, welches mit Wasser wenig mischbar oder nicht mischbar ist und in welchem Atorvastatincalcium unlöslich oder praktisch unlöslich ist, wobei ungefähr das gleiche oder ein höheres Volumen als das in Schritt e) hinzugegebene Wasservolumen eingesetzt wird;
g) Optionales Durchführen einer Mischoperation und Trennen der zwei Phasen;
h) Neutralisieren der wässrigen Phase;
i) Umwandeln der Dihydroxycarbonsäureform von Atorvastatin in eine pharmazeutisch akzeptable Salzform; und
j) Ausbilden eines Präzipitats des Atorvastatins, das in die pharmazeutisch akzeptable Salzform umgewandelt worden ist.

2. Das Verfahren nach Anspruch 1, wobei eine Lösung mit der intermediären Verbindung mit der folgenden Formel (II) in Schritt a) bereitgestellt wird: 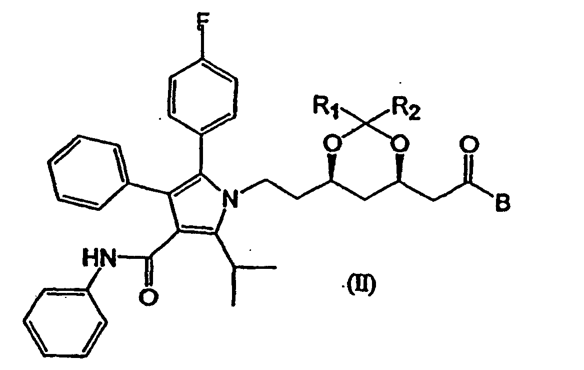 worin R₁ und R₂ unabhängigerweise Wasserstoff, Alkyl aus ein bis drei Kohlenstoffatomen oder Phenyl sind, oder R₁ und R₂ als (-CH₂)ₙ- zusammengenommen sind, wobei n gleich 4 oder 5 ist,
B gleich das folgende ist:
a) O-R₃, wobei R₃ das folgende ist
- ein geradkettiges oder verzweigtes Alkyl aus einem bis acht Kohlenstoffatomen (R₃ ist tert-Butyl, tert-Amyl oder α,α-Dimethylbenzyl), oder
- eine drei- bis sechsgliedrige Cycloalkylgruppe,
b) eine Gruppe der folgenden Formel: 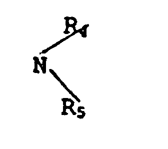
worin R₄ und R₅ unabhängigerweise Alkyl mit ein bis zehn Kohlenstoffatomen, Cycloalkyl aus drei bis sieben Kohlenstoffatomen, Aryl oder Aralkyl sind, oder R₄ und R₅ zusammen eine vier- bis sechsgliedrige Kohlenwasserstoffbrückengruppe ausbilden, die optional ein oder mehrere Heteroatome enthält und optional mit einem Alkyl aus einem bis vier Kohlenstoffatomen substituiert ist.

3. Das Verfahren nach Anspruch 1, wobei eine Lösung mit der intermediären Verbindung mit der folgenden Formel (III) in Schritt a) bereitgestellt wird:

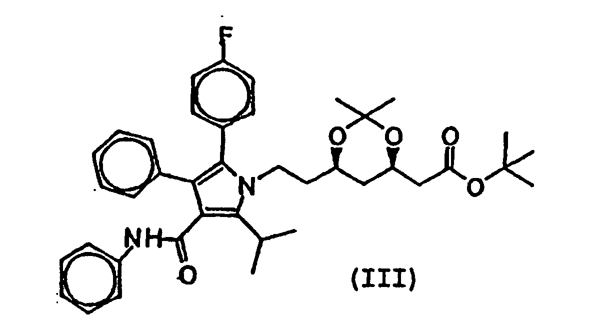

4. Das Verfahren nach irgendeinem der Ansprüche 1 bis 3, wobei das Entschützen der Dihydroxygruppe in Schritt b) durch Zugeben einer Säure und durch Halten oder Mischen der Lösung bei einer Temperatur von 5 bis 40°C durchgeführt wird.

5. Das Verfahren nach irgendeinem der Ansprüche 1 bis 3, wobei das Entschützen der Carbonsäureschutzgruppe in Schritt c) durch Zugeben einer Base zur Einstellung des pH der Lösung in einem Bereich von 8 bis 13 und durch Halten oder Mischen der Lösung bei einer Temperatur von 5 bis 40°C durchgeführt wird.

6. Das Verfahren nach irgendeinem der Ansprüche 1 bis 3, wobei in Schritt d) die Lösung auf 15 bis 50 % des anfänglichen Volumens konzentriert wird.

7. Das Verfahren nach irgendeinem der Ansprüche 1 bis 3, wobei Wasser in Schritt e) in der 0,6 bis 3-fachen Menge, relativ zu dem Volumen der konzentrierten Lösung, hinzugegeben wird.

8. Das Verfahren nach irgendeinem der Ansprüche 1 bis 3, wobei Wasser in Schritt e) in der 0,6 bis 1,5-fachen Menge, relativ zu dem Volumen der konzentrierten Lösung, hinzugegeben wird.

9. Das Verfahren nach irgendeinem der Ansprüche 1 bis 3, wobei das Lösungsmittel in Schritt f) in einer Menge vom 1 bis 5-fachen des in Schritt e) im Voraus hinzugegebenen Wasservolumens hinzugegeben wird.

10. Das Verfahren nach irgendeinem der Ansprüche 1 bis 3, wobei das Neutralisieren der wässrigen Phase in Schritt h) durch Zugeben einer Säure zu der wässrigen Phase durchgeführt wird, um ihren pH auf einen Bereich von 6,5 bis 8 einzustellen.

11. Das Verfahren nach irgendeinem der Ansprüche 1 bis 3, wobei die Umwandlung in Schritt e) durch Erwärmen der neutralisierten wässrigen Lösung auf eine Temperatur von 30 bis 40°C und dann durch Zugeben einer wässrigen Lösung des korrespondierenden Salzes, die auf 30 bis 40°C vorerwärmt worden ist, durchgeführt wird.

12. Das Verfahren nach irgendeinem der Ansprüche 1 bis 3 oder 11, wobei nach der Zugabe eines korrespondierenden Salzes die Lösung unter einer Mischoperation bei einer Temperatur im Bereich von 10 bis 30°C gehalten wird.

13. Das Verfahren nach irgendeinem der Ansprüche 1 bis 3, 11 oder 12, wobei das Salz ein Calciumsalz ist.

14. Das Verfahren nach irgendeinem der Ansprüche 1 bis 3, wobei der Präzipitationsschritt j) das Einstellen der Temperatur der Lösung in einen Bereich von 2 bis 15°C umfasst, um ein Präzipitat aus nicht-kristallinem Atorvastatin in der pharmazeutisch akzeptablen Salzform zu erzielen.

15. Das Verfahren nach irgendeinem der Ansprüche 1 bis 3, wobei der Präzipitationsschritt j) das Zugeben eines organischen Lösungsmittels umfasst, welches mit Wasser mischbar ist und in welchem Atorvastatin praktisch unlöslich oder unlöslich ist.

16. Das Verfahren nach irgendeinem der Ansprüche 1 bis 3, wobei der Präzipitationsschritt j) das Konzentrieren der Lösung umfasst.

17. Das Verfahren nach irgendeinem der Ansprüche 1 bis 3 und 14 bis 16, welches einen weiteren Schritt k) umfasst, und zwar das Abfiltrieren des ausgebildeten Präzpitats, das Waschen des Präzipitats mit Wasser und das Trocknen des Präzipitats, um nicht-kristallines Atorvastatincalcium zu erzeugen.

18. Ein Verfahren zur Herstellung von Atorvastatincalcium in einer nicht-kristallinen Form, welches das Folgende umfasst:
- Bereitstellen von Atorvastatin in der Form eines Lactons in einem nicht-hydroxylischen Lösungsmittel;
- Durchführen einer Reaktion zur Öffnung des Lactonrings; und dann
- Durchführen der in Anspruch 1 definierten Schritte d) bis j).

19. Das Verfahren nach Anspruch 18, wobei der Lactonring durch Zugeben einer Base und Erwärmen der Lösung auf eine Temperatur von 40 bis 60°C geöffnet wird.

20. Das Verfahren nach Anspruch 18, wobei irgendeiner der Verfahrensschritte, wie sie in den Ansprüchen 6 bis 17 definiert sind, in Verbindung mit den Schritten d) bis f) und h) bis k) durchgeführt werden.

21. Ein Verfahren zur Herstellung einer pharmazeutischen Formulierung mit Atorvastatin in einer nicht-kristallinen Form, umfassend das Herstellen von Atorvastatin in einer nicht-kristallinen Form gemäß Anspruch 1 oder Anspruch 18 und das Vermischen von diesem mit einem pharmazeutisch akzeptablen Träger.

22. Das Verfahren nach Anspruch 21, wobei das nichtkristalline Atorvastatin in der Calciumsalzform hergestellt wird.

## Revendications

1. Procédé de préparation d'atorvastatine sous une forme non cristalline, qui comprend les étapes consistant à :
a) préparer une solution contenant un composé intermédiaire ayant la formule (I) suivante dans un solvant non hydroxylique : 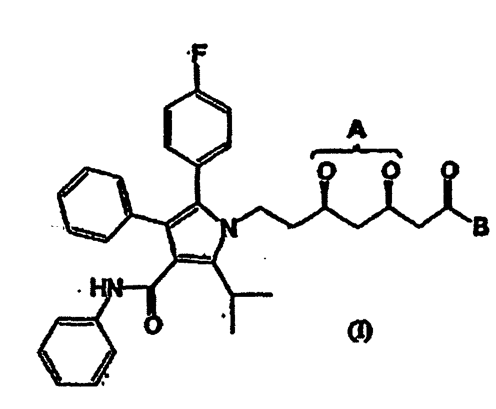 dans laquelle A désigne un groupe de protection commun ou des groupes de protection séparés pour le groupe dihydroxy et B désigne un groupe de protection d'acide carboxylique ;
b) déprotéger le groupe dihydroxy ;
c) déprotéger le groupe de protection d'acide carboxylique ;
dans lequel l'ordre des étapes b) et c) peut être inversé ;
d) concentrer la solution à environ la moitié du volume initial ou moins ;
e) ajouter de l'eau en excédent par rapport au volume de la solution concentrée ;
f) ajouter, en utilisant un volume environ égal ou supérieur au volume d'eau ajouté à l'étape e), un solvant qui est légèrement miscible ou non miscible à l'eau et dans lequel l'atorvastatine-calcium est insoluble ou pratiquement insoluble ;
g) éventuellement, procéder à une opération de mélange, et séparer les deux phases ;
h) neutraliser la phase aqueuse ;
i) convertir la forme acide dihydroxycarboxylique de l'atorvastatine en une forme sel pharmaceutiquement acceptable ; et
j) former un précipité de l'atorvastatine convertie en ladite forme sel pharmaceutiquement acceptable.

2. Procédé selon la revendication 1, dans lequel une solution contenant le composé intermédiaire ayant la formule (II) suivante est préparée dans l'étape a) : 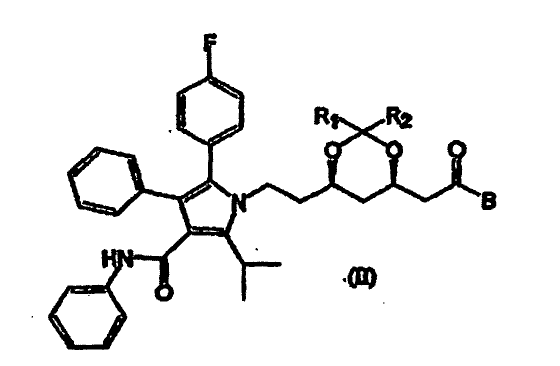 dans laquelle R₁ et R₂ sont, indépendamment, un atome d'hydrogène, un groupe alkyle ayant de un à trois atomes de carbone, ou phényle, ou R₁ et R₂ sont pris ensemble et forment (-CH₂)ₙ- où n est 4 ou 5,
B est
a) O-R₃ où R₃ est
- un groupe alkyle à chaîne droite ou à chaîne ramifiée ayant de un à huit atomes de carbone (R₃ est tert-butyle, tert-amyle, ou α,α-diméthylbenzyle), ou
- un groupe cycloalkyle ayant de trois à six chaînons,
b) un groupe de formule 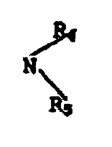
dans laquelle R₄ et R₅ sont, indépendamment, un groupe alkyle ayant de un à dix atomes de carbone, cycloalkyle ayant de trois à sept atomes de carbone, aryle ou aralkyle, ou R₄ et R₅ forment ensemble un groupe de liaison hydrocarboné à quatre à six chaînons contenant éventuellement un ou plusieurs hétéroatomes et étant éventuellement substitué par un groupe alkyle ayant de un à quatre atomes de carbone.

3. Procédé selon la revendication 1, dans lequel une solution contenant le composé intermédiaire ayant la formule (III) suivante est préparée dans l'étape a) :

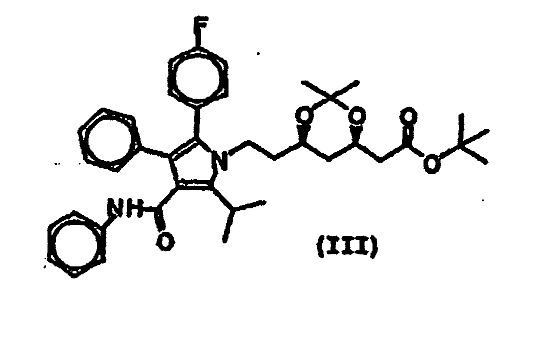

4. Procédé selon l'une quelconque des revendications 1 à 3, dans lequel la déprotection du groupe dihydroxy dans l'étape b) est réalisée en ajoutant un acide et en maintenant ou en mélangeant la solution à une température de 5 à 40°C.

5. Procédé selon l'une quelconque des revendications 1 à 3, dans lequel la déprotection du groupe de protection d'acide carboxylique dans l'étape c) est réalisée en ajoutant une base pour ajuster le pH de la solution dans une plage de 8 à 13 et en maintenant ou en mélangeant la solution à une température de 5 à 40°C.

6. Procédé selon l'une quelconque des revendications 1 à 3, dans lequel dans l'étape d) la solution est concentrée à 15 à 50 % du volume initial.

7. Procédé selon l'une quelconque des revendications 1 à 3, dans lequel l'eau est ajoutée dans l'étape e) à raison de 0,6 à 3 fois par rapport au volume de la solution concentrée.

8. Procédé selon l'une quelconque des revendications 1 à 3, dans lequel l'eau est ajoutée dans l'étape e) à raison de 0,6 à 1,5 fois par rapport au volume de la solution concentrée.

9. Procédé selon l'une quelconque des revendications 1 à 3, dans lequel ledit solvant est ajouté dans l'étape f) en une quantité de 1 à 5 fois le volume d'eau précédemment ajouté dans l'étape e).

10. Procédé selon l'une quelconque des revendications 1 à 3, dans lequel la neutralisation de la phase aqueuse dans l'étape h) est réalisée en ajoutant un acide à la phase aqueuse pour ajuster son pH dans la plage de 6,5 à 8.

11. Procédé selon l'une quelconque des revendications 1 à 3, dans lequel la conversion dans l'étape i) est réalisée en chauffant la solution aqueuse neutralisée à une température de 30 à 40°C, puis en ajoutant une solution aqueuse du sel correspondant préchauffée à 30 à 40°C.

12. Procédé selon l'une quelconque des revendications 1 à 3, ou 11, dans lequel, après l'addition d'un sel correspondant, la solution est soumise à une opération de mélange à une température dans la plage de 10 à 30°C.

13. Procédé selon l'une quelconque des revendications 1 à 3, 11 ou 12, dans lequel le sel est un sel de calcium.

14. Procédé selon l'une quelconque des revendications 1 à 3, dans lequel l'étape de précipitation j) comprend l'ajustement de la température de la solution dans une plage de 2 à 15°C pour obtenir un précipité d'atorvastatine non cristalline sous la forme d'un sel pharmaceutiquement acceptable.

15. Procédé selon l'une quelconque des revendications 1 à 3, dans lequel l'étape de précipitation j) comprend l'addition d'un solvant organique qui est miscible à l'eau et dans lequel l'atorvastatine est pratiquement insoluble ou insoluble.

16. Procédé selon l'une quelconque des revendications 1 à 3, dans lequel l'étape de précipitation j) comprend la concentration de la solution.

17. Procédé selon l'une quelconque des revendications 1 à 3, 14 à 16, qui comprend une étape supplémentaire k) qui consiste à séparer le précipité formé par filtration, à rincer le précipité avec de l'eau, et à sécher le précipité pour obtenir l'atorvastatine-calcium non cristalline.

18. Procédé de préparation l'atorvastatine-calcium sous une forme non cristalline, qui comprend les étapes consistant à :
- préparer l'atorvastatine sous la forme lactone dans un solvant non hydroxylique ;
- mettre en oeuvre une réaction pour ouvrir le cycle lactone ; puis
- effectuer les étapes telles que définies par les étapes d) à j) exposées dans la revendication 1.

19. Procédé selon la revendication 18, dans lequel le cycle lactone est ouvert en ajoutant une base et en chauffant la solution jusqu'à une température de 40 à 60°C.

20. Procédé selon la revendication 18, dans lequel l'une quelconque des étapes du procédé définies dans les revendications 6 à 7 concernant les étapes d)-f) et h)-k) est exécutée.

21. Procédé de préparation d'une formulation pharmaceutique contenant de l'atorvastatine sous une forme non cristalline, comprenant les étapes consistant à préparer l'atorvastatine sous une forme non cristalline selon la revendication 1 ou la revendication 18 et à la mélanger avec un véhicule pharmaceutiquement acceptable.

22. Procédé selon la revendication 21, dans lequel une atorvastatine non cristalline sous forme de sel de calcium est préparée.
